Europäisches Patentamt

European Patent Office

Office européen des brevets

(11) Publication number: **0 197 729**

A2

# EUROPEAN PATENT APPLICATION

(21) Application number: 86302331.3

(22) Date of filing: 27.03.86

(51) Int. Cl.⁴: **G 01 N 33/53**
//G01N33/74

(30) Priority: 01.04.85 IE 830/85

(43) Date of publication of application:
15.10.86 Bulletin 86/42

(84) Designated Contracting States:
AT BE CH DE FR GB IT LI LU NL SE

(71) Applicant: Kelly, Donald Patrick
25 Sydney Parade
Ballsbridge Dublin(IE)

(71) Applicant: Cleere, William Francis
Gortard
Clarinbridge Galway(IE)

(72) Inventor: Kelly, Donald Patrick
25 Sydney Parade
Ballsbridge Dublin(IE)

(72) Inventor: Cleere, William Francis
Gortard
Clarinbridge Galway(IE)

(74) Representative: Goldin, Douglas Michael et al,
J.A. KEMP & CO. 14, South Square Gray's Inn
London WC1R 5EU(GB)

(54) Enzyme immunoassay method.

(57) In an enzyme immunoassay method in which the degree of binding of a component of an antigen-antibody reaction with an enzyme conjugate is measured by incubating the two together in a plurality of wells 11 in a microtitreplate 10 and subsequently measuring the optical density provided by the action of the enzyme on a substrate, the antigen/antibody is introduced into each well coated on a respective moulded plastics insert 12. The inserts are preferably of downwardly tapering hollow conical shape.

FIG. 3

This invention relates to an enzyme immunoassay (EIA) method using a microtitreplate (MTP).

An MTP is a known tray-like article having a plurality of wells which are usually arranged in a rectangular array, typically having 8 rows by 12 columns, and which are usually made by moulding from a transparent plastics material such as transparent polystyrene. In conventional use the tray including the wells is coated with an antigen or antibody either directly or indirectly and used to measure the degree of binding of the antibody with an enzyme conjugate by introducing the latter into the wells of the coated tray, incubating the two together in the tray, and subsequently measuring the optical density (OD) provided by the action of the enzyme on a substrate (reagent for colour development). The OD of each well is therefore a measure of the degree of binding in the well.

However, we have found that under nominally identical conditions a typical antibody-coated MTP shows trends and localised "highs" and "lows" in the degree of antibody binding in the wells.

This can be demonstrated by coating an MTP with antibody and measuring the amount of antibody in each well by its ability to bind an enzyme conjugate, the conditions in each well being made identical as far as possible. In this case the OD readings will be a measure of the amount of antibody in each well, and variations of the OD reading will reflect variations in antibody binding from well-to-well.

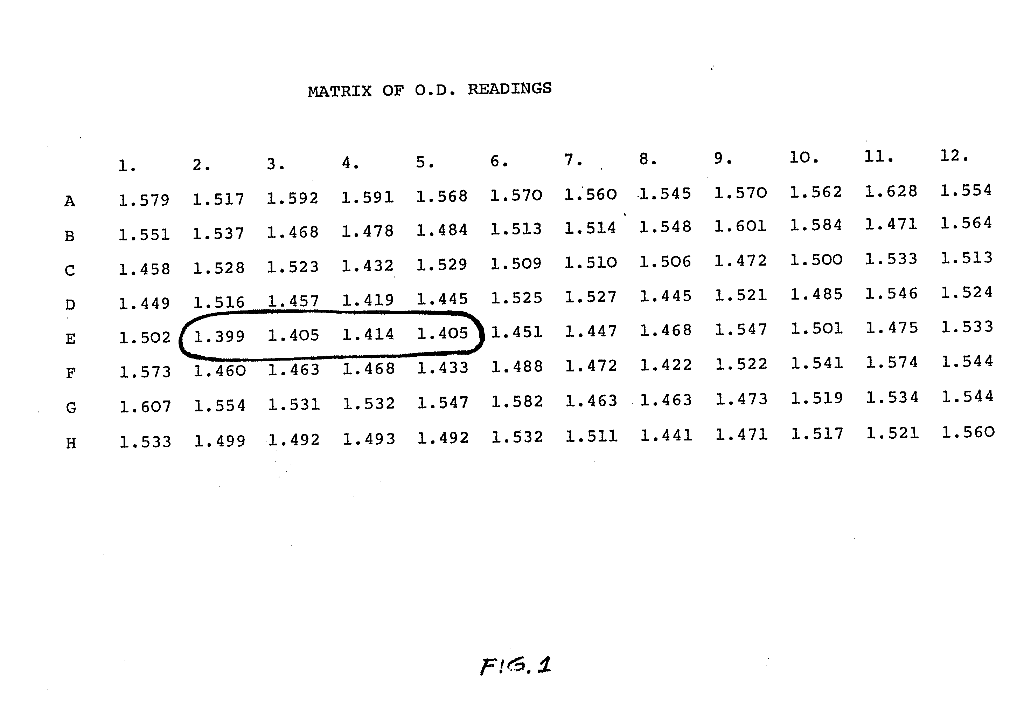

A typical example is shown in figure 1, where the letters A to H refer to the rows and the numbers 1 to 12 refer to the columns of an 8 x 12 matrix of wells in a 96-well MTP.

It will be observed that the general trend is for the OD to drop from row A to row E, the trend is reversed as one goes to row G, and finally there is a drop at row H. As regards colums there is a drop-rise-drop-rise pattern from left to right. Finally, an area of localised lows is found at wells E2 to E5.

It would seem that these trends inherently result from the moulding process by which the plate is made. This is clearly undesirable since OD variations will be partially accounted for by the different binding characteristics of antibody in the wells thus reducing the accuracy of the assay.

Accordingly, the present invention provides an EIA method in which the degree of binding of a component of an antigen-antibody reaction with an enzyme conjugate is measured by incubating the two together in a plurality of wells in an MTP and subsequently measuring the optical density provided by the action of the enzyme on a substrate, wherein the antigen or antibody is introduced into each well coated on a respective moulded plastics insert.

Thus, of the two roles played by the MTP in the conventional technique, i.e. (i) solid phase for antigen/antibody binding and (ii) incubation vessel, the inserts substitute for role (i) while the MTP continues to serve for role (ii).

The main advantage of the invention is that, assuming the inserts are randomly located in the wells, local "highs" and "lows" and general trends would be eliminated or substantialy reduced, giving a substantial improvement of the assay. Overall variation in binding between any two inserts should be less than the variation between any two wells in a preformed plate, since in the latter the wells are formed in different sub-moulds of the main plate mould, whereas all the inserts may be formed on the same mould or, if formed in batches in sub-moulds, they may be sorted so that all inserts from one sub-mould are kept together.

A further advantage of the invention is that the inserts can reduce incubation time if a shape which gives a large surface area (and hence more binding) to volume ratio is used. More binding means more enzyme conjugate bound and less time is required for colour development. It will be appreciated that in the conventional technique the surface area to volume ratio is fixed for a given plate.

In practice inserts could be used individually for small numbers of tests, that is, they can be inserted individually by hand into the wells of the MTP. However, for large numbers of tests the inserts may be first assembled in a holding frame to form an array corresponding to the array of wells in the MTP, and then introduced substantially simultaneously into the respective wells by lowering the frame towards the MTP. For this purpose each insert would be provided with at least one upwardly extending support member by which the insert can be gripped by the frame while leaving the insert proper freely depending below the frame for insertion into the MTP.

The advantage of this arrangement is that the incubation time can be precisely controlled simply by lowering and raising the frame at the desired time, and the incubation time will be substantially the same for all wells. The elimination of the abovementioned local "highs" and "lows" and general trends would also be achieved in this case by randomly loading the holding frame with the inserts.

The inserts may be adapted to be moved progressively from one row of wells to one or more subsequent rows of wells during the course of a particular assay as described hereinbelow in greater detail. For example, the inserts may be moved from a first row of wells in which the immunochemical reaction takes place and hence to a second row of wells in which the enzymatic reaction takes place and optical density measurements are taken.

Where the inserts are to be loaded individually by hand, it is preferred that they are of hollow open-ended construction so that they do not have to be removed from the wells prior to measurement of the OD which is conventionally performed by directing light vertically through the wells. Open-ended inserts of downwardly tapering conical shape have been found suitable for this purpose. The use of inserts of a conical shape provides two binding surfaces for the antibody compared to the one surface (the inside wall of the well) when the MTP alone is used. Inserts of the same open-ended hollow conical shape may also be used where they are held in a frame, although if the frame does not permit the passage of light down through each well the frame will have to be removed prior to OD measurement.

The inserts may be made of any plastics material which binds antigen/antibody such as polypropylene, and it is immaterial whether the material is opaque or not as the insert will not be in the path of the light when OD measurement takes place (either because it is removed or because it is hollow and open-ended). A particular advantage of polypropylene, although not limited thereto, is that it has the ability to be dry-coated with antibody. In the latter case it is possible to pre-assemble inserts dry-coated with antibody into the wells of an MTP and supply the assembly ready for use to the end user who simply needs to add the enzyme conjugate and any other reagent(s) according to the particular assay being conducted. The invention therefore also provides an MTP having inserts dry-coated with antigen/antibody accommodated in respective wells thereof.

Antigen/antibody may be bound directly to the insert or, alternatively, indirect binding may be used. For example, the inserts may be coated directly with antibody and then a layer of antigen bound to the antibody coated on the inserts, if direct binding of the particular antigen is difficult or impossible to achieve. Indirect binding of antibody may also be used, although this would not normally be the case.

An embodiment of the invention will now be described, by way of example, with reference to the accompanying drawings, in which:

Figure 1, previously described, is a table which shows the variation in antibody binding in the wells of a conventional MTP,

Figure 2 is a cross-sectional perspective view of part of a conventional MTP,

Figure 3 is a cross-sectional perspective view of part of a conventional MTP but having inserts in the wells thereof for practising the present invention,

Figure 4 is a perspective view of a single insert for use with a holding frame, and

Figure 5 is a labelled diagrammatic top view of an MTP which is used in association with a description of a detailed embodiment of the invention using the inserts of Figure 4.

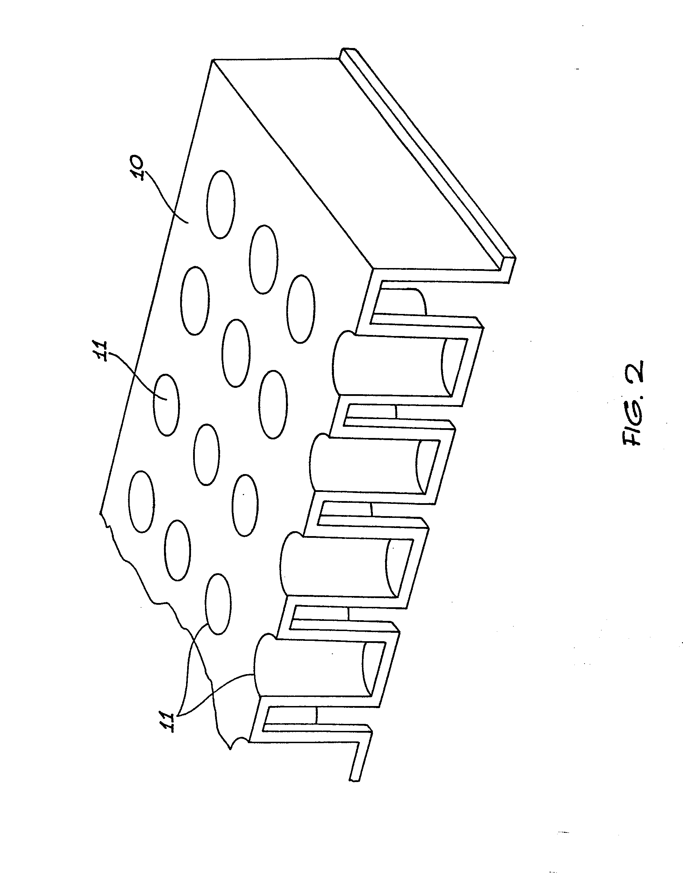

Referring to figure 2, a conventional MTP 10 is formed as a one-piece moulding of transparent polystyrene and contains an 8 x 12 rectangular array of parallel cylindrical wells 11. As mentioned above, such a plate has the disadvantage that the moulding technique conventionally used in the manufacture of the plates inherently produces non-uniform antibody binding characteristics across the area of the plate.

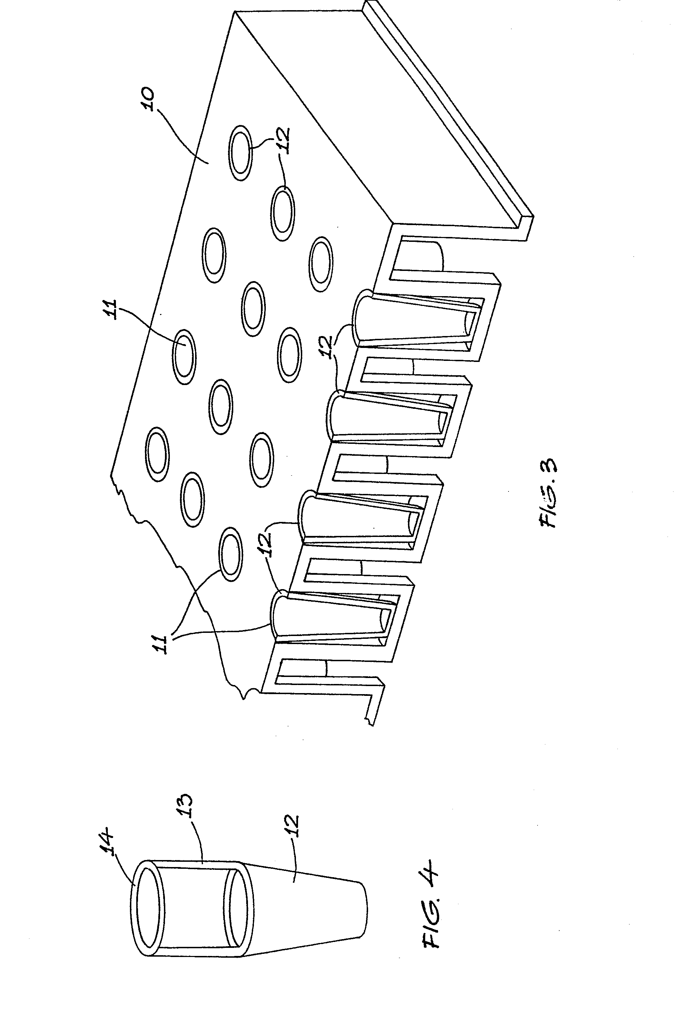

Thus, in accordance with the embodiment of the invention shown in figure 3, inserts 12 previously coated with antibody are inserted into each well 11. The inserts 12 are of hollow open-ended conical form which taper downwardly into the wells, each insert being a snug fit at the top of the well and having its axis coincident with the axis of the well. The hollow form of the inserts permits a reading of optical density to be taken without removing the inserts from the wells.

The inserts are moulded from a plastics material which binds antibody, and they are preferably made of polypropylene as this material can be dry-coated with antibody and therefore plates and inserts can be supplied to the user in ready-assembled form as illustrated in figure 3.

However, the inserts may be inserted into the wells in wet or dry coated form by the user, and this may be done before or after the enzyme conjugate is added. In the example shown in figure 3, if the assembly is not supplied ready-assembled as shown, the user must place each insert individually into the wells. To assist in the insertion and removal of the inserts 12 shown in figure 3, these may project in part above the surrounding plate, for example they may have an annular rim at the top end which rests on the top surface of the plate 10, or they may be provided with an upward projection similar to one of the members 13 of figure 4 now to be described.

To avoid the insertion of each insert 12 individually into its respective well, figure 4 illustrates a form of insert which is adapted to be fitted into a holding frame (not shown) along with a plurality of similar inserts. The main body part of the insert is the same as that shown in figure 3, and is similarly referenced, but a pair of members 13 extend upwardly from diametrically opposite points on the upper rim of the main body 12 and terminate in a ring 14. The rings 14 of all the inserts are gripped by the holding frame in such positions relative to the frame as to form an array corresponding in configuration to the wells 11 of the plate 10, the bodies 12 of all the inserts depending from the same side of the frame. Thus, as described above, all the coated inserts may be introduced into and removed from the respective wells at the same time by lowering and raising the frame as a whole towards or away from the plate 10.

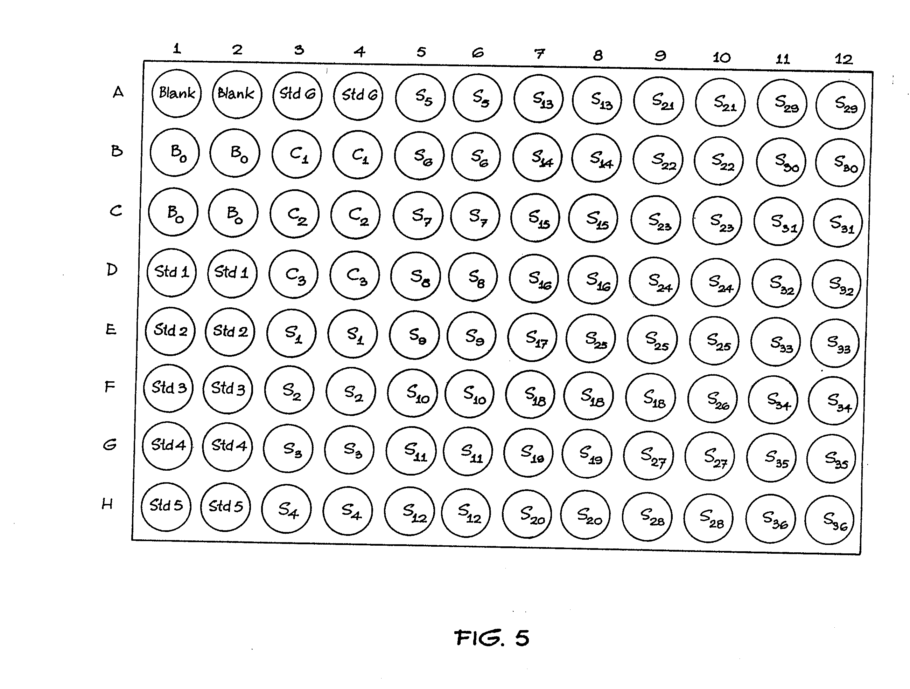

An embodiment of the invention using inserts in a frame will now be described with reference to figure 5. The particular EIA being performed in this case is that of progesterone in milk using inserts pre-coated with anti-progesterone serum and assembled in an 8 x 12 matrix in the frame. Referring to figure 5, the steps of the method are as follows:

(1)    Add 200 1 of enzyme-progesterone conjugate (Prog-HRP) to each well in the MTP (except wells A1 and A2).

(2)    Add 10 1 milk progesterone (standards, controls and samples) to appropriate wells in the plate.

(3)    Mix.

(4)    Place frame of inserts in the wells. This begins the first stage of the reaction. Prog-HRP will bind to the antibody coated inserts in the absence of milk progesterone (Bo wells). In the presence of increasing amounts of milk progesterone (STD1 to STD6) less and less Prog-HRP will bind.

(5)    After 1 hour empty the plate and wash the inserts (still in the wells) with a MTP washer. Remove the frame of inserts.

(6)    To each well in the plate add 150 1 of buffer-substrate (i.e. reagent for colour development).

(7)    Replace the frame of inserts. This begins the reaction which leads to colour development.

(8)   After 30 mins.  Remove the frame of inserts.  Colour development ceases.

(9)   The colour developed in each well is read on an EIA reader.

(10)  A curve of progesterone concentration versus colour is prepared for the standards.  By reference to this curve the progesterone concentration of a sample may be measured from its colour.

The removal of the frame at step 8 implies that the frame used does not permit the passage of light down through the wells.  However, it will be appreciated that the rings 14 would permit this with a suitably designed holding frame, so that with a suitable design of frame the inserts would not need to be removed for OD measurement.

In a further preferred embodiment the microtitreplate comprises a cuboid transparent polystyrene block containing rows of cylindrical wells, generally in multiples of three, together with a holding frame to which is attached a plurality of similar inserts and which inserts are moved by moving the holding frame in the course of an assay progressively to the successive rows of wells.  The well inserts are coated with antigen/antibody to the substance being determined and are supplied in a first row of wells containing a stabilising solution.  In the case of the progesterone in milk assay referred to above the inserts are coated with progesterone antiserum and the stabilising solution is glycerol based.  The second or middle row of wells contain dilute, ready-to-use enzyme conjugate solution and to this is added a predetermined amount of the sample to be tested.  In the case of the progesterone in milk assay one drop of milk would be added to the wells.  The antigen/antibody coated well inserts are removed from the first row of wells, washed clear and immersed into the wells containing the enzyme conjugate plus sample wherein the biochemical binding reactions of enzyme immunoassay occur. After an appropriate time sufficient to enable the immunochemical reaction to proceed to completion, the well inserts are removed, washed and inserted into the third row of wells which contain a buffer-substrate mixture to which the user will have added a predetermined amount of chromogen solution shortly before. The conjugate bound to the inserts during the incubation in the second row of wells causes development of colour in the complete buffer-substrate mixture. The intensity of the colour produced, relative to one or more supplied control(s) indicates the presence or the amount of the substance being determined. The advantage of this embodiment is its simplicity in use, since manipulations are cut to the absolute minimum. Besides the block and inserts any kit comprising same and, indeed, the end user, will require only a bottle of chromogen for addition to the buffer-substrate in the third row of wells and a control vial or vials of various controls to be added to the row of conjugate wells at the same time as the samples to the analysed.

It is to be understood that the invention is by no means restricted to the use of hollow inserts nor conical inserts, and any shape of insert which will fit within the wells 11 may be used. Furthermore, the inserts of figure 4, or any other inserts designed for use with a holding frame, can be inserted manually into the respective wells if desired without the use of a frame.

CLAIMS:-

1.    An enzyme immunoassay (EIA) method in which the degree of binding of a component of an antigen-antibody reaction with an enzyme conjugate is measured by incubating the two together in a plurality of wells in a microtitreplate (MTP) and subsequently measuring the optical density provided by the action of the enzyme on a substrate, wherein the antigen or antibody is introduced into each well coated on a respective moulded plastics insert.

2.    An EIA method according to claim 1, wherein each insert is of hollow open-ended construction oriented in its respective well to permit light to be shone vertically through the well without obstruction by the insert.

3.    An EIA method according to claim 2, wherein the inserts are of downwardly tapering conical shape.

4.    An EIA method according to any preceding claim, wherein each insert projects at least in part above the surrounding MTP.

5.    An EIA method according to claim 4, wherein the inserts are assembled in a holding frame and inserted simultaneously into the MTP by lowering the frame.

6.    An EIA method according to any preceding claim, wherein the inserts are dry-coated with antigen or antibody.

7.    An EIA method according to claim 6, wherein the inserts are inserted in the wells before the enzyme conjugate.

8.   An EIA method according to claim 7, wherein the inserts are of polypropylene.

9.   A microtitreplate having a plurality of wells each containing a respective moulded plastics insert dry-coated with an antigen or antibody.

10.   A microtitreplate according to claim 9, wherein the inserts are of polypropylene.

MATRIX OF O.D. READINGS

|   | 1. | 2. | 3. | 4. | 5. | 6. | 7. | 8. | 9. | 10. | 11. | 12. |
|---|---|---|---|---|---|---|---|---|---|---|---|---|
| A | 1.579 | 1.517 | 1.592 | 1.591 | 1.568 | 1.570 | 1.560 | 1.545 | 1.570 | 1.562 | 1.628 | 1.554 |
| B | 1.551 | 1.537 | 1.468 | 1.478 | 1.484 | 1.513 | 1.514 | 1.548 | 1.601 | 1.584 | 1.471 | 1.564 |
| C | 1.458 | 1.528 | 1.523 | 1.432 | 1.529 | 1.509 | 1.510 | 1.506 | 1.472 | 1.500 | 1.533 | 1.513 |
| D | 1.449 | 1.516 | 1.457 | 1.419 | 1.445 | 1.525 | 1.527 | 1.445 | 1.521 | 1.485 | 1.546 | 1.524 |
| E | 1.502 | 1.399 | 1.405 | 1.414 | 1.405 | 1.451 | 1.447 | 1.468 | 1.547 | 1.501 | 1.475 | 1.533 |
| F | 1.573 | 1.460 | 1.463 | 1.468 | 1.433 | 1.488 | 1.472 | 1.422 | 1.522 | 1.541 | 1.574 | 1.544 |
| G | 1.607 | 1.554 | 1.531 | 1.532 | 1.547 | 1.582 | 1.463 | 1.463 | 1.473 | 1.519 | 1.534 | 1.544 |
| H | 1.533 | 1.499 | 1.492 | 1.493 | 1.492 | 1.532 | 1.511 | 1.441 | 1.471 | 1.517 | 1.521 | 1.560 |

FIG. 1

0197729

FIG. 2

FIG. 4

FIG. 3

0197729

FIG. 5